# EUROPEAN PATENT APPLICATION

(11) **EP 4 230 731 A1**
(43) Date of publication of application: **23.08.2023**
(21) Application number: 20957327.8
(22) Date of filing: 23.10.2020
(51) Int. Cl.: C12N 5/0793, A61K 31/4745, A61P 25/00

(54) **COMPOSITION AND METHOD FOR TRANSDIFFERENTIATING NON-NEURONAL CELLS INTO NEURONS**

(30) Priority: 14.10.2020 CN 202011097347
(71) Applicant: Institute Of Zoology, Chinese Academy Of Sciences, Beijing 100101 (CN); Beijing Institute for Stem Cell and Regenerative Medicine, Beijing 100101 (CN)
(72) Inventor: LI, Wei, Beijing 100101 (CN); ZHOU, Qi, Beijing 100101 (CN); HE, Zhengquan, Beijing 100101 (CN)
(74) Representative: Lavoix
(86) International application number: PCT/CN2020/123227
(87) International publication number: WO 2022/077549

(57) **Abstract**

Provided are a composition for inducing cell transdifferentiation and use thereof in inducing the transdifferentiation of non-neuronal cells into neurons. The composition comprises a myosin inhibitor, and an isoxazole compound and/or a derivative thereof. Further provided is a method for inducing the transdifferentiation of non-neuronal cells into neurons, comprising: culturing the non-neuronal cells in an induction culture solution comprising a myosin inhibitor, and then culturing them in a mature culture solution comprising a myosin inhibitor, and an isoxazole compound and/or a derivative thereof, so as to obtain mature neurons. Also provided is a method for transdifferentiating non-neuronal cells within a subject into neurons, comprising administering to the subject an effective amount of a myosin inhibitor, and an isoxazole compound and/or a derivative thereof.

## Description

### TECHNICAL FIELD

The present application relates to the field of biotechnology, and in particular to a composition and method for transdifferentiation of non-neuronal cells into neurons.

### BACKGROUND ART

Neurodegenerative diseases are diseases caused by the loss of neurons and/or their myelin sheaths, which worsen and become dysfunctional over time. Common neurodegenerative diseases include Parkinson's disease (PD), Alzheimer's disease (AD), amyotrophic lateral sclerosis (ALS), Huntington's disease (HD) and different types of spinocerebellar ataxia (SCA), epilepsy, cerebral apoplexy (also known as stroke), brain injury and spinal cord injury. Promoting neuron regeneration is a key link and an important means in the treatment of such diseases. How to achieve simple and efficient neuron regeneration has been a hotspot given attention for a long time. At present, the prior art has disclosed some methods for transdifferentiation of non-neuronal cells into neurons.

Patent Document 1 (CN103849601B) discloses a method for inducing transdifferentiation of fibroblasts into neuronal cells and use thereof. The transformation process adopts a retrovirus system, and miRNA-302/367 cluster, miRNA-9 and miRNA-124 are stably and efficiently overexpressed in human fibroblasts, thereby regulating a series of biochemical reactions in cells, and transdifferentiating fibroblasts into neuronal cells.

Patent Document 2 (CN106337037A) discloses a pharmaceutical composition for inducing direct transformation of fibroblasts into neural cells and use thereof. The transdifferentiation between non-lineage neural cells is achieved by the combination of small molecule compounds without exogenous genes. The disclosed pharmaceutical composition for inducing direct transformation of fibroblasts into neural cells comprises the main medicinal components of VPA, CHIR-99021, RepSox, Forskolin, SP600125, Go6983 and Y-27632.

Patent Document 3 (CN110283788A) discloses a method for inducing the reprogramming of spinal cord astrocytes into motor neurons, which selects 7 kinds of small molecule drugs SB431542, LDN193189, RA, bFGF, Purmorphamine, Forskolin and VPA, wherein the reprogramming of astrocytes is induced in vitro by small molecule drugs, thereby inducing the reprogramming of rat astrocytes into motor neurons.

In the prior art, in vitro overexpression of transcription factors can achieve the transdifferentiation of non-neuronal cells (e.g., fibroblasts or astrocytes) into neurons, but these methods have not been able to achieve safe application in vivo. In addition, since chemical small molecules have the advantages of convenient cell processing, good permeability, no immunogenicity, and easy local or systemic administration, a number of prior art studies have achieved transdifferentiation of human fibroblasts into neurons through complex combinations of multiple small molecules; however, transdifferentiation of the cells into neurons is difficult to achieve in vivo due to low transdifferentiation efficiency and too many small molecules.

### Prior Art Literatures:

Patent document 1, CN103849601B announcement text
Patent document 2, CN106337037A publication text
Patent document 3, CN110283788A publication text

### SUMMARY OF THE APPLICATION

In order to realize the safe transdifferentiation of non-neuronal cells into neurons in vivo and improve the transdifferentiation efficiency, the present application provides a method for efficient neuronal transdifferentiation mediated by a combination of simple small molecule compounds. The present application provides a simpler and easier way for transdifferentiation of human or animal non-neuronal cells into neurons, and achieves pioneering and unexpected technical effects. The technical solutions of the present application are as follows:
The present application provides a composition for inducing cell transdifferentiation, comprising:
a myosin inhibitor, and
an isoxazole compound and/or a derivative thereof.

The present application provides use of a composition comprising a myosin inhibitor, and an isoxazole compound and/or a derivative thereof in inducing cell transdifferentiation.

Preferably, the transdifferentiation is to induce the transdifferentiation of non-neuronal cells into neurons.

The present application provides use of a composition comprising a myosin inhibitor, and an isoxazole compound and/or a derivative thereof in the preparation of a medicament for treating a neurodegenerative disease.

The present application provides a method for inducing transdifferentiation of non-neuronal cells into neurons, characterized in that it comprises treating the non-neuronal cells with a myosin inhibitor, and an isoxazole compound and/or a derivative thereof.

Preferably, the method for inducing transdifferentiation of non-neuronal cells into neurons provided herein comprises: culturing the non-neuronal cells in an induction culture solution for 1-7 days, and then culturing them in a mature culture solution for 7-45 days, preferably 21-45 days.

The present application provides a culture medium for inducing transdifferentiation of non-neuronal cells into neurons, comprising an induction culture solution and a mature culture solution.

Preferably, the induction culture solution comprises a myosin inhibitor;

Preferably, the induction culture solution comprises N2B27 culture solution and a myosin inhibitor, wherein the N2B27 culture solution is prepared by firstly mixing DMEM/F12 and Neurobasal at a ratio of 1:1, and then adding N2 cell culture additive, B27 cell culture additive, β-mercaptoethanol, Glutamax, insulin and penicillin-streptomycin.

Preferably, the mature culture solution comprises a myosin inhibitor, and an isoxazole compound and/or a derivative thereof.
Preferably, the mature culture solution comprises: a myosin inhibitor, an isoxazole compound and/or a derivative thereof, N2B27 culture solution, neurotrophic factor and forskolin; wherein the N2B27 culture solution is prepared by firstly mixing DMEM/F12 and Neurobasal at a ratio of 1:1, and then adding N2 cell culture additive, B27 cell culture additive, β-mercaptoethanol, Glutamax, insulin and penicillin-streptomycin;
preferably, the neurotrophic factor comprises: neurotrophin-3, brain-derived neurotrophic factor and glial cell-derived neurotrophic factor;
preferably, the mature culture solution comprises: a myosin inhibitor, an isoxazole compound and/or a derivative thereof, and the N2B27 culture solution;
preferably, the mature culture solution consists of: a myosin inhibitor, an isoxazole compound and/or a derivative thereof, and the N2B27 culture solution;
preferably, the mature culture solution comprises: a myosin inhibitor, an isoxazole compound and/or a derivative thereof, the N2B27 culture solution, neurotrophin-3, brain-derived neurotrophic factor and glial cell-derived neurotrophic factor;
preferably, the mature culture solution consists of: a myosin inhibitor, an isoxazole compound and/or a derivative thereof, the N2B27 culture solution, neurotrophin-3, brain-derived neurotrophic factor and glial cell-derived neurotrophic factor.

The present application provides a method for transdifferentiating non-neuronal cells into neurons in a subject, comprising administering to the subject an effective amount of a myosin inhibitor, and an isoxazole compound and/or a derivative thereof.

Preferably, the method comprises: administering to the subject an effective amount of a myosin inhibitor, and an isoxazole compound and/or a derivative thereof by intraperitoneal injection.

Preferably, the method comprises: culturing the non-neuronal cells in an induction culture solution and a mature culture solution in sequence, then injecting the cultured non-neuronal cells into the body, and finally administering to the subject an effective amount of a myosin inhibitor, and an isoxazole compound and/or a derivative thereof by intraperitoneal injection.

Preferably, the method comprises: culturing the non-neuronal cells in an induction culture solution for 1-7 days and subsequently in a mature culture solution for 5-10 days, then injecting the cultured non-neuronal cells into the body, and finally administering to the subject an effective amount of a myosin inhibitor, and an isoxazole compound and/or a derivative thereof by intraperitoneal injectionfor 14 or more consecutive days.

The present application provides a method for treating a neurodegenerative disease in a subject, comprising administering to the subject an effective amount of a myosin inhibitor, and an isoxazole compound and/or a derivative thereof.

Preferably, the neurodegenerative disease includes: Parkinson's disease, Alzheimer's disease, amyotrophic lateral sclerosis, Huntington's disease, spinocerebellar ataxia, epilepsy, stroke, brain injury, and spinal cord injury.

The present application provides neurons obtained by the above method for inducing transdifferentiation of non-neuronal cells into neurons.

The present application provides an article of manufacture or kit for transdifferentiating non-neuronal cells into neurons, wherein the article of manufacture or kit comprises an induction culture solution and a mature culture solution, wherein
the induction culture solution comprises a myosin inhibitor;
the mature culture solution comprises a myosin inhibitor, and an isoxazole compound and/or a derivative thereof.

The present application provides use of a myosin inhibitor in the promotion of neuronal morphogenesis and the initiation of neural fate.

The present application provides use of an isoxazole compound or a derivative thereof in the promotion of efficient expression of neuron genes.

Preferably, the myosin inhibitor is (-)-Blebbistatin, and/or (-)-Blebbistatin O-Benzoate.

Preferably, the isoxazole compound and/or a derivative thereof has a structure represented by the following formula (I), 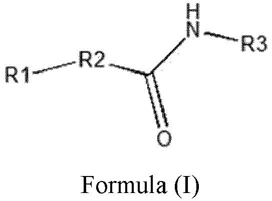 in formula (I), R1 group is any one selected from the group consisting of: thienyl, furanyl, pyrrolyl, phenyl and pyridyl; R2 group is any one selected from the group consisting of: isoxazolyl, isothiazolyl, pyrazolyl, oxazoly, thiazolyl and imidazolyl, R3 group is any one selected from the group consisting of: methyl, ethyl, cyclopropyl, cyclobutyl and cyclopentyl; wherein the linking site of R1 group is any carbon atom, the two linking sites of the R2 group are two meta-position carbon atoms, and the linking site of the R3 group is any carbon atom.

Preferably, the isoxazole compound and/or a derivative thereof is any one or more selected from the group consisting of: isoxazole 9 (ISX9), N-methyl-5-phenylisoxazole-3-carboxamide (ISX-PCA), N,5-dimethylisoxazole-3-carboxamide, N-methyl-5-(pyridin-4-yl)isoxazole-3-carboxamide, N-methyl-5-phenylisothiazole-3-carboxamide, N-methyl-5-phenyl-1H-pyrazole-3-carboxamide, N-methyl-2-phenyloxazole-4-carboxamide, N-methyl-2-phenylthiazole-4-carboxamide, N-methyl-2-phenyl-1H-imidazole-4-carboxamide, N-methyl-5-(thiophen-2-yl)isoxazole-3-carboxamide, 5-(furan-2-yl)-N-methylisoxazole-3-carboxamide, N-methyl-2-(thiophen-2-yl)-1,3-thiazole-4-carboxamide.

Preferably, the non-neuronal cells are fibroblasts or astrocytes.

### Effects of the Application

According to the present application, with a combined treatment by using a composition comprising a myosin inhibitor, and an isoxazole compound and/or a derivative thereof, the transdifferentiation of non-neuronal cells (e.g., fibroblasts or astrocytes) into neurons can be efficiently achieved in vitro, thereby providing a new cell source for the acquisition of neurons in vitro in regenerative medicine; and the transdifferentiation of the cells into neurons in vivo can also be achieved by intraperitoneal injection of the composition, thereby achieving neuron regeneration to help to treat a neurodegenerative disease. It has not been reported that achieving cell fate change to obtain neurons by the method according to the present application. Compared with the previously reported cell fate regulation method, the application of this method is simpler, only two simple small molecules are combined for the treatment, and no over expression regulation of specific genes is needed; the cell fate can be changed only by changing the culture substrate for the cells, and it can be efficiently performed in vitro and in vivo, so as to achieve simple and efficient neuron regeneration, and provide a new approach for the treatment of a neurodegenerative disease in vivo caused by aging and pathological damage.

The characteristics of the present application lie in that: 1. the operation is simple, firstly the non-neuronal cells are added in an induction culture solution comprising a myosin inhibitor for culture, and then the cells are continuously cultured in a mature culture solution comprising a myosin inhibitor, and an isoxazole compound and/or a derivative thereof, so that the cell fate can be changed; 2. it is efficient and fast, and in the process of the transdifferentiation of non-neuronal cells into neurons by combined treatment with a myosin inhibitor, and an isoxazole and/or a derivative thereof, obvious neuronal morphology can appear on day 1-7; 3. universality, the method has universal applicability in the process of transdifferentiation of different types of initiating cells from different species into neurons; 4. compared with the prior art, the method according to the present application has stronger in vivo inducibility; 5. safety, the method according to the present application has higher safety than traditional viral vector-mediated genetics methods; 6. controllability, compared with a combination of multiple complex small molecules, the slow-release system of two simple small molecules is more feasible for mediated transdifferentiation and more convenient for metering control.

### BRIEF DESCRIPTION OF THE DRAWINGS

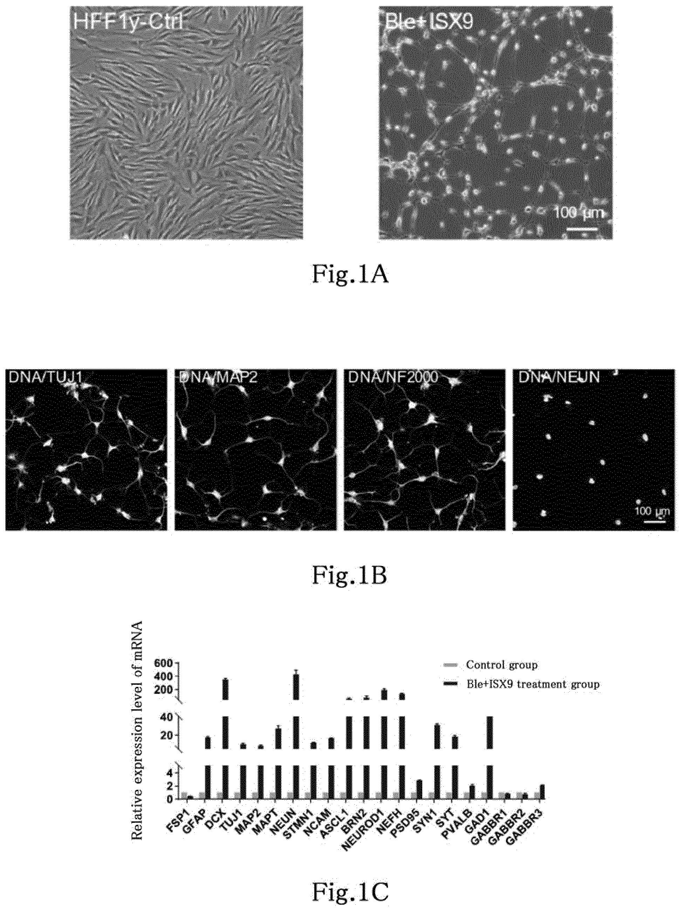

Fig. 1A is a micrograph of human foreskin fibroblasts (HFF1y, Beijing Stem Cell Bank) (left), and a micrograph of induced neurons (right) obtained by: firstly culturing the human foreskin fibroblasts (HFF1y, Beijing Stem Cell Bank) in an induction culture solution comprising a myosin II inhibitor (-)-Blebbistatin for 7 days, and then culturing them in a mature culture solution comprising (-)-Blebbistatin and an isoxazole compound ISX9 for 21 days.
Fig. 1B is a graph showing the staining results of neuronal markers for the induced neurons of Fig. 1A (right).
Fig. 1C shows the gene expressions of the neuronal markers in induced neurons obtained by: firstly culturing the human foreskin fibroblasts (HFF1y, Beijing Stem Cell Bank) of Fig. 1A (left) in an induction culture solution comprising (-)-Blebbistatin for 7 days, and then culturing them in a mature culture solution comprising (-)-Blebbistatin and an isoxazole compound ISX9 for 38 days.

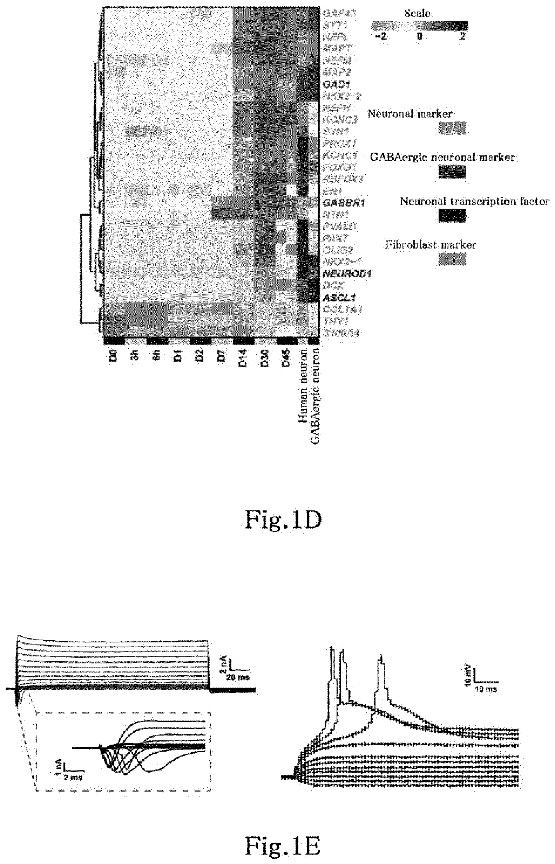

Fig. 1D shows the expression heat map of nerve-related genes in the process of inducing neurons as follows: firstly culturing the human foreskin fibroblasts (HFF1y, Beijing Stem Cell Bank) of Fig. 1A (left) in an induction culture solution for 7 days, and then culturing them in a mature culture solution for 38 days.
Fig. 1E is a graph showing the results of a patch clamp experiment on the induced neurons in Fig. 1A (right); particularly, the panel on the left is a detected current diagram of the sodium and potassium, and the panel on the right is an evoked action potential diagram.

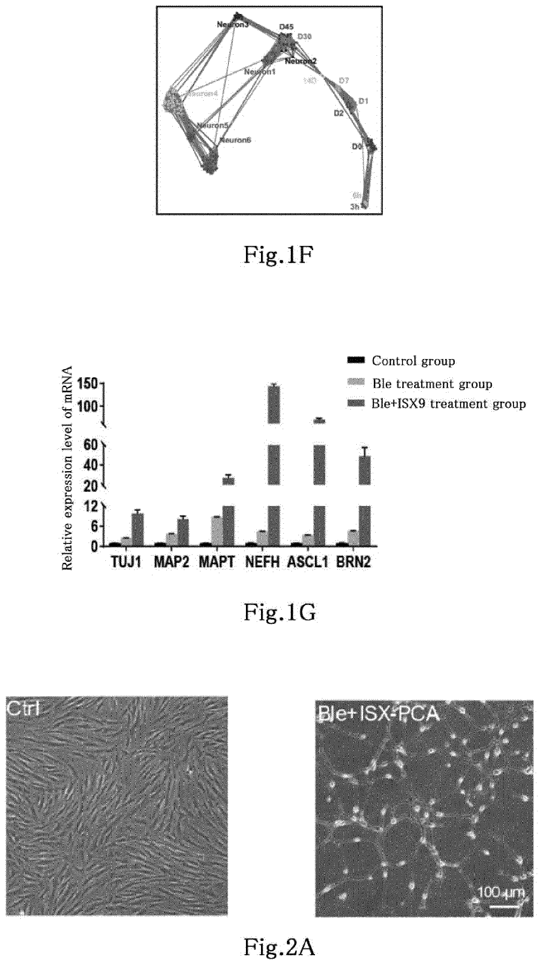

Fig. 1F shows a comparison of the sequencing results of neurons in vivo and the sequencing results of single cell obtained by the whole process of: firstly culturing the human foreskin fibroblasts (HFF1y, Beijing Stem Cell Bank) of Fig. 1A (left) in an induction culture solution comprising (-)-Blebbistatin for 7 days, and then culturing them in a mature culture solution comprising (-)-Blebbistatin and an isoxazole compound ISX9 for 38 days (the 3rd hour, the 6th hour, day 1, day 2, day 7, day 24, day 30, and day 45).
Fig. 1G shows a comparison of gene expressions of neuronal markers in the induced neurons from the human foreskin fibroblasts (HFF1y, Beijing Stem Cell Bank) of Fig. 1A (left) of the following groups: induced neurons obtained by firstly culturing the cells in an induction culture solution comprising (-)-Blebbistatin for 7 days, and then culturing them in a mature culture solution comprising (-)-Blebbistatin and an isoxazole compound ISX9 for 45 days; induced neurons obtained by firstly culturing the cells in an induction culture solution comprising (-)-Blebbistatin for 7 days, and then culturing them in a mature culture solution without (-)-Blebbistatin and an isoxazole compound ISX9 for 45 days; and induced neurons of blank control group.
Fig. 2A is a micrograph of human foreskin fibroblasts (HFF1y, Beijing Stem Cell Bank) (left), and a micrograph of induced neurons (right) obtained by: firstly culturing the human foreskin fibroblasts (HFF1y, Beijing Stem Cell Bank) in an induction culture solution comprising (-)-Blebbistatin for 7 days, and then culturing them in a mature culture solution comprising (-)-Blebbistatin and ISX-PCA (a derivative of the isoxazole compound ISX9) for 21 days.

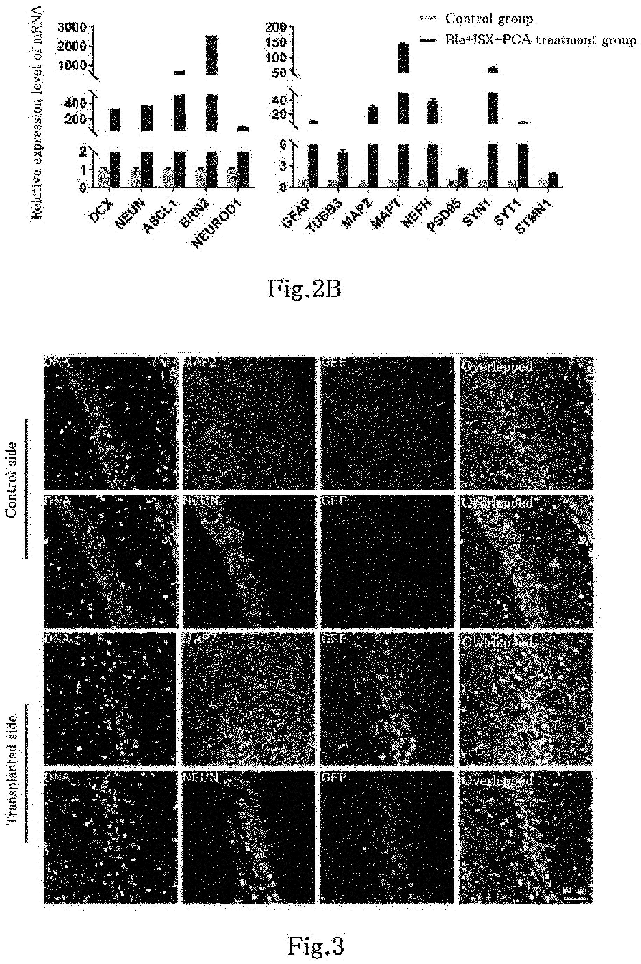

Fig. 2B shows the gene expressions of the neuronal markers in induced neurons obtained by: firstly culturing the human foreskin fibroblasts (HFF1y, Beijing Stem Cell Bank) of Fig. 2A (left) in an induction culture solution comprising (-)-Blebbistatin for 7 days, and then culturing them in a mature culture solution comprising (-)-Blebbistatin and ISX-PCA (a derivative of the isoxazole compound ISX9) for 45 days.
Fig. 3 shows staining results of neuronal transdifferentiation of the cells obtained by: firstly culturing the human foreskin fibroblasts (HFF1y, Beijing Stem Cell Bank)in an induction culture solution comprising (-)-Blebbistatin, and culturing them in a mature culture solution comprising (-)-Blebbistatin and an isoxazole compound ISX9, then transplanting the cells into the body and intraperitoneally injecting (-)-Blebbistatin and the isoxazole compound ISX9 (marking the transplanted cells with GFP).

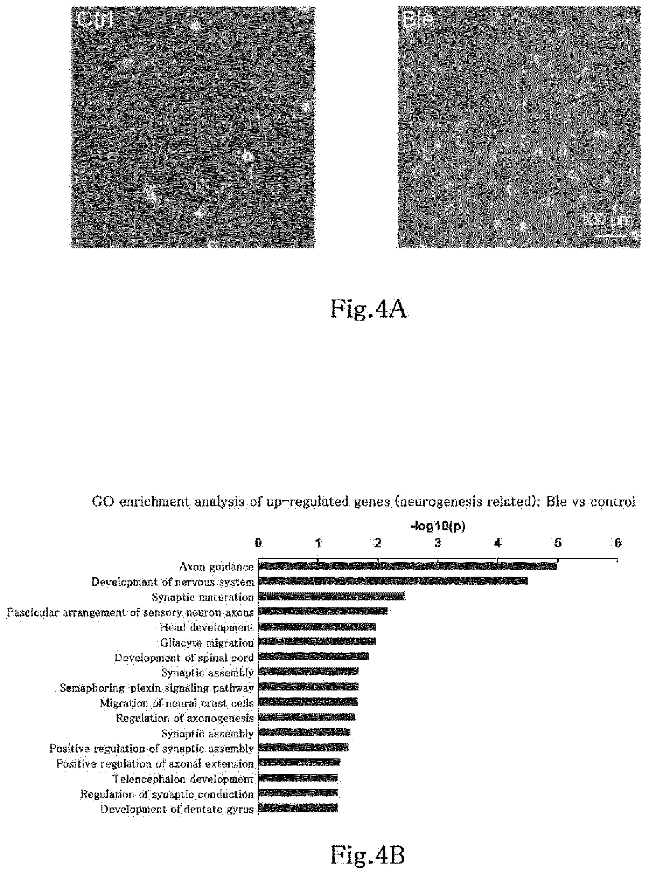

Fig. 4A is a micrograph of human foreskin fibroblasts (HFF1y, Beijing Stem Cell Bank) (left), and a micrograph of induced neurons (right) obtained by: firstly culturing the human foreskin fibroblasts (HFF1y, Beijing Stem Cell Bank) in an induction culture solution comprising (-)-Blebbistatin for 7 days.
Fig. 4B shows the results of GO clustering analysis on the induced neurons in Fig. 4A (right) after RNA-seq.

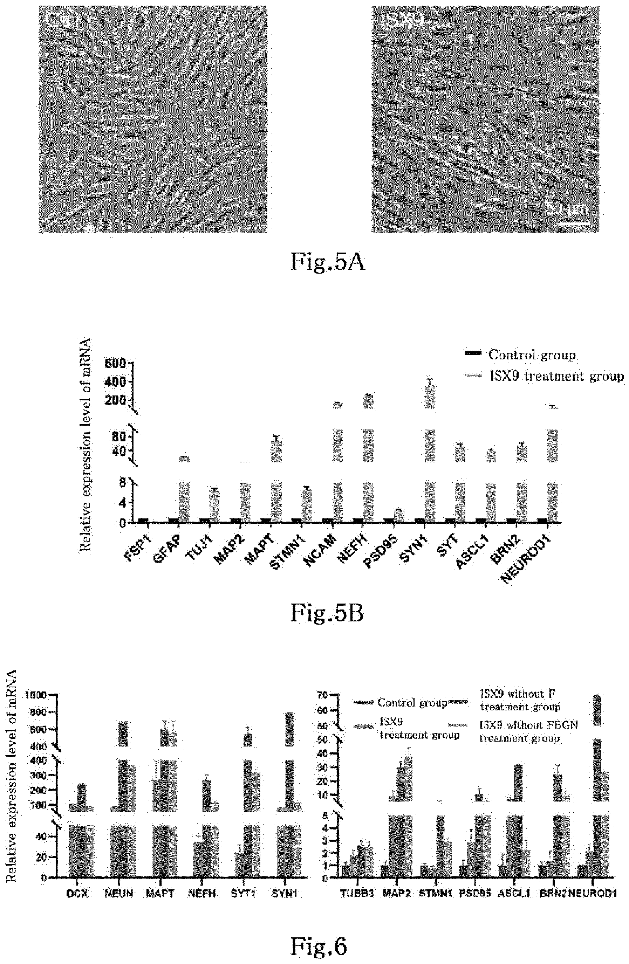

Fig. 5A is a micrograph of human foreskin fibroblasts (HFF1y, Beijing Stem Cell Bank) (left), and a micrograph of induced cells (right) obtained by: firstly culturing the human foreskin fibroblasts (HFF1y, Beijing Stem Cell Bank) in an induction culture solution without a myosin II inhibitor (-)-Blebbistatin for 1 day, and then culturing them in a mature culture solution comprising an isoxazole compound ISX9 and without (-)-Blebbistatin for 6 days.
Fig. 5B shows gene expressions of neuronal markers for the induced cells of Fig. 5A (right).
Fig. 6 shows gene expressions of the canonical neuronal markers for the induced cells obtained by: firstly culturing the cells of Fig. 5A (left) in an induction culture solution for 1 day, then culturing them in a neural cell mature culture solution removing forskolin or removing neurotrophin-3, brain-derived neurotrophic factor, glial cell-derived neurotrophic factor and forskolin simultaneously for 6 days (wherein F stands for forskolin, FBGN stands for a mixture of forskolin, brain-derived neurotrophic factor, glial cell-derived neurotrophic factor and neurotrophin-3).

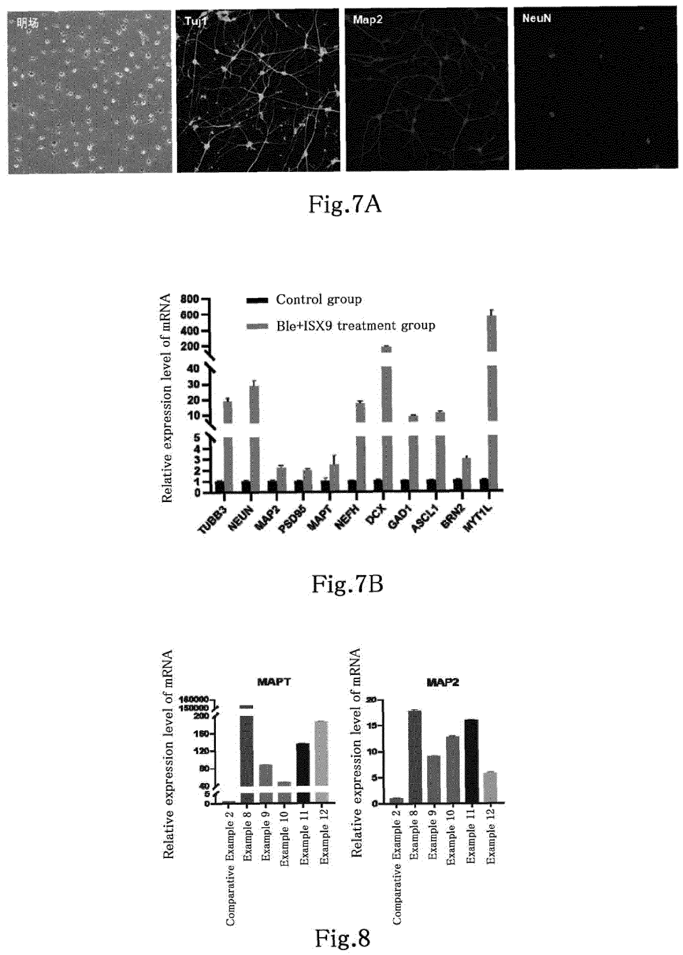

Fig. 7A shows morphology of the induced neurons obtained by firstly culturing the mouse astrocytes in an induction culture solution comprising (-)-Blebbistatin for 1 day, then culturing them in a mature culture solution comprising (-)-Blebbistatin and an isoxazole compound ISX9 for 13 days; and staining results of neuronal markers for the induced neurons.
Fig. 7B shows the gene expressions of neuronal markers for the induced neurons of Fig. 7A.
Fig. 8 is a comparison result on gene expressions of neuronal markers for the neurons obtained by induction culture with different induction culture solutions and mature culture solutions.

### DETAILED DESCRIPTION OF THE APPLICATION

The embodiments of the present application will be illustrated and described in detail below through specific examples, but the following contents should not be construed as any limitation to the present application.

The terms used herein have the following meanings:
High glucose DMEM: a high glucose DMEM medium (dulbecco's modified eagle medium, DMEM), i.e., a commercial medium comprising various glucose and amino acids, developed on the basis of MEM medium.

N2B27 culture solution: a well-defined cell culture solution comprising N2 cell culture additive and B27 cell culture additive in combination of a mixture of DMEM/F12 basal medium and Neurobasal basal medium at a ratio of 1:1. According to the different cells to be cultured, other components in the N2B27 culture solution other than the above-mentioned components are also different. It is reported that it is beneficial to the differentiation of mouse embryonic stem cells into the neural cell. The N2B27 culture solution of the present application is prepared by mixing DMEM/F12 and Neurobasal at a ratio of 1:1, and then adding N2 cell culture additive, B27 cell culture additive, β-mercaptoethanol, Glutamax, insulin and penicillin-streptomycin.

DMEM/F12: a commercial basal medium solution prepared by mixing DMEM medium and F12 medium at a ratio of 1:1, which is suitable for the cultivation at clonal density.

Neurobasal: a commercial basal medium beneficial for neural cell culture.

Glutamax: a cell culture additive that directly replaces L-glutamine in a cell culture medium.

Penicillin-streptomycin: penicillin and streptomycin are two antibiotics commonly used in cell culture to prevent bacterial contamination during cell culture.

N2 cell culture additive: a commercial serum-free cell culture additive.

B27 cell culture additive: a commercial serum-free cell culture additive.

Neurotrophin 3: neurotrophin (NT) is a kind of protein molecule produced by innervated tissues (such as muscle) and astrocytes, and is necessary for neuron growth and survival. Neurotrophin-3 (NT-3) is a kind of neurotrophic factor, in the nervous system it is mainly distributed in the dorsal root ganglion, spinal cord, brain stem, cerebellum and hippocampus etc., and it can maintain sympathetic, sensory, basal forebrain cholinergic and motor neuron survival.

Brain-derived neurotrophic factor: a kind of neurotrophic factor, which is the most abundant neurotrophic factor in the body. It acts by combining with TrkB (tyrosine kinase B), and is distributed in the central nervous system, peripheral nervous system, endocrine system, bone and cartilage tissue etc., but is mainly expressed in the central nervous system, with the highest content in the hippocampus and cortex.

Glial cell-derived neurotrophic factor: it can support the survival of midbrain dopaminergic neurons in vitro, and improve the survival rate of dopaminergic neurons and the density of nerve endings in various animal models of Parkinson's disease, thereby ameliorating the symptoms.

GABA: short for gamma aminobutyric acid, is an important neurotransmitter in the central nervous system, as an inhibitory neurotransmitter it has an impact on learning, memory, and sleep.

GABAN: short for GABAergic neuron, which refers to the part of nerve cells that mainly use GABA as a transmitter.

The present application provides a composition for inducing cell transdifferentiation, which comprises:
a myosin inhibitor, and
an isoxazole compound and/or a derivative thereof.

The derivative in this application refers to a derivative of an isoxazole compound.

In a particular embodiment, the transdifferentiation is to induce the transdifferentiation of non-neuronal cells into neurons, and the non-neuronal cells are fibroblasts or astrocytes; the myosin inhibitor is (-)- Blebbistatin and/or (-)-Blebbistatin O-Benzoate.
(-)-Blebbistatin used herein, abbreviated as Ble, has the structure represented by formula (II). 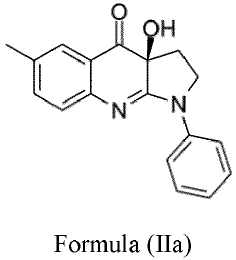
(-)-Blebbistatin O-Benzoate used herein, abbreviated as Ble-OB, has the structure represented by formula (IIb).

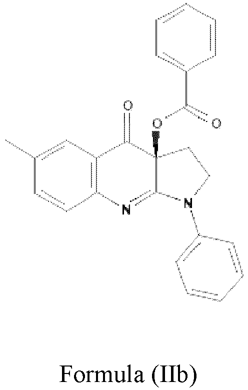

The present application also provides use of a composition comprising a myosin inhibitor, and an isoxazole compound and/or a derivative thereof in inducing cell transdifferentiation.

The present application also provides use of a composition comprising a myosin inhibitor, and an isoxazole compound and/or a derivative thereof in the preparation of a medicament for treating a neurodegenerative disease.

In a particular embodiment, the neurodegenerative disease includes, but is not limited to: Parkinson's disease, Alzheimer's disease, amyotrophic lateral sclerosis, Huntington's disease, spinocerebellar ataxia, epilepsy, stroke, brain injury, and spinal cord injury.

The present application provides a method for inducing transdifferentiation of non-neuronal cells into neurons in vitro, which comprises treating non-neuronal cells with a myosin inhibitor, and an isoxazole compound and/or a derivative thereof.

In a particular embodiment, the method comprises: firstly culturing the non-neuronal cells in an induction culture solution for 1-7 days, optionally 1 day, 2 days, 3 days, 4 days, 5 days, 6 days or 7 days, and then culturing in a mature culture solution for 7-45 days, preferably 21-45 days, more preferably 30-45 days, optionally 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 31 days, 32 days, 33 days, 34 days, 35 days, 36 days, 37 days, 38 days, 39 days, 40 days, 41 days, 42 days, 43 days, 44 days, or 45 days, etc.; wherein the induction culture solution comprises a myosin inhibitor, and the mature culture solution comprises a myosin inhibitor, and an isoxazole compound and/or a derivative thereof.

The present application also provides a method for transdifferentiating non-neuronal cells into neurons in a subject, which comprises administering to the subject an effective amount of a myosin inhibitor, and an isoxazole compound and/or a derivative thereof.

In a particular embodiment, the method comprises: firstly culturing the non-neuronal cells in an induction culture solution for 1-7 days, optionally 1 day, 2 days, 3 days, 4 days, 5 days, 6 days, 7 days etc., subsequently culturing in a mature culture solution for 5-10 days, optionally 5 days, 6 days, 7 days, 8 days, 9 days, 10 days etc., preferably 7 days, and then injecting the cultured non-neuronal cells into the body and finally administering an effective amount of a myosin inhibitor, and an isoxazole compound and/or a derivative thereof to the subject by intraperitoneal injection for 14 or more consecutive days (optionally 14 days, 15 days, 16 days, 17 days, 18 days, 19 days, etc.).

The present application also provides a method for treating a neurodegenerative disease in a subject, wherein it comprises administering to the subject an effective amount of a myosin inhibitor, and an isoxazole compound and/or a derivative thereof.

The present application also provides a medium for inducing the transdifferentiation of non-neuronal cells into neurons, wherein it comprises an induction culture solution and a mature culture solution.

In a particular embodiment, the induction culture solution comprises: N2B27 culture solution and a myosin inhibitor, and the mature culture solution comprises: N2B27 culture solution, neurotrophic factor, forskolin, a myosin inhibitor, and an isoxazole compound and/or a derivative thereof; wherein the N2B27 culture solution is prepared by mixing DMEM/F12 and Neurobasal at a ratio of 1:1, then adding N2 cell culture additive, B27 cell culture additive, β-mercaptoethanol, Glutamax, insulin and penicillin-streptomycin.

In a particular embodiment, the neurotrophic factor comprises: neurotrophin-3, brain-derived neurotrophic factor, and glial cell-derived neurotrophic factor.

In a particular embodiment, the mature culture solution comprises: a myosin inhibitor, an isoxazole compound and/or a derivative thereof, and the N2B27 culture solution, excluding neurotrophic factor and forskolin.

In a particular embodiment, the mature culture solution consists of a myosin inhibitor, an isoxazole compound and/or a derivative thereof, and the N2B27 culture solution.

In a particular embodiment, the mature culture solution comprises: a myosin inhibitor, an isoxazole compound and/or a derivative thereof, the N2B27 culture solution, neurotrophin-3, brain-derived neurotrophic factor, and glial cell-derived neurotrophic factor, excluding forskolin.

In a preferred embodiment, the mature culture solution consists of: a myosin inhibitor, an isoxazole compound and/or a derivative thereof, the N2B27 culture solution, neurotrophin-3, brain-derived neurotrophic factor, and glial cell-derived neurotrophic factor.

In a particular embodiment, the concentration of the neurotrophin-3 is 0-25ng/mL, optionally 0ng/mL, 1ng/mL, 2ng/mL, 3ng/mL, 4ng/mL, 5ng/mL, 6ng /mL, 7ng/mL, 8ng/mL, 9ng/mL, 10ng/mL, 11ng/mL, 12ng/mL, 13ng/mL, 14ng/mL, 15ng/mL, 16ng/mL, 17ng/mL, 18ng/mL, 19ng/mL, 20ng/mL, 21ng/mL, 22ng/mL, 23ng/mL, 24ng/mL, 25ng/mL etc., the concentration of the brain-derived neurotrophic factor is 0-25ng/mL, optionally 0ng/mL mL, 1ng/mL, 2ng/mL, 3ng/mL, 4ng/mL, 5ng/mL, 6ng/mL, 7ng/mL, 8ng/mL, 9ng/mL, 10ng/mL, 11ng/mL, 12ng/mL, 13ng/mL, 14ng/mL, 15ng/mL, 16ng/mL, 17ng/mL, 18ng/mL, 19ng/mL, 20ng/mL, 21ng/mL, 22ng/mL, 23ng/mL, 24ng/mL, 25ng/mL etc., the concentration of the glial cell-derived neurotrophic factor is 0-25ng/mL, optionally 0ng/mL, 1ng/mL, 2ng/mL, 3ng/mL, 4ng/mL, 5ng/mL, 6ng/mL, 7ng/mL, 8ng/mL, 9ng/mL, 10ng/mL, 11ng/mL, 12ng/mL, 13ng/mL, 14ng/mL, 15ng/mL, 16ng/mL, 17ng/mL, 18ng/mL, 19ng/mL, 20ng/mL, 21ng/mL, 22ng/mL, 23ng/mL, 24ng/mL, 25ng/mL etc., the concentration of the forskolin is 0-20µM, optionally 0ng/mL, 1ng/mL, 2ng/mL, 3ng/mL, 4ng/mL, 5ng/mL, 6ng/mL, 7ng/mL, 8ng/mL, 9ng/mL, 10ng/mL, 11ng/mL, 12ng/mL, 13ng/mL, 14ng/mL, 15ng /mL, 16ng/mL, 17ng/mL, 18ng/mL, 19ng/mL, 20ng/mL etc. Particularly, the concentration is a final concentration of each of the three different nutrient factors in the mature culture solution.

In a particular embodiment, the concentration of the myosin inhibitor in the induction culture solution is 5-25 µM, optionally 5 µM, 10 µM, 15 µM, 20 µM, 21 µM, 22 µM, 23 µM, 24 µM, or 25 µM. Particularly, the concentration is the final concentration of the myosin inhibitor in the induction culture solution for treating the non-neuronal cells.

In a particular embodiment, the concentration of the myosin inhibitor in the mature culture solution is 0-25 µM, optionally 0 µM, 5 µM, 10 µM, 15 µM, 20 µM, or 25 µM; the concentration of the isoxazole compound or a derivatives thereof is 20-50 µM, optionally 20 µM, 22 µM, 24 µM, 26 µM, 28 µM, 30 µM, 32 µM, 34 µM, 36 µM, 40 µM, 42 µM, 44 µM, 46 µM, 48 µM, 50 µM etc. Particularly, the concentrations are respectively the final concentrations of the myosin inhibitor, and the isoxazole compound or a derivative thereof in the induction culture solution for treating the non-neuronal cells.

In a particular embodiment, the non-neuronal cells are firstly cultured in a basal medium, and then cultured in an induction culture solution and a mature culture solution sequentially.

In a particular embodiment, the basal medium is prepared with high glucose DMEM plus 10% fetal bovine serum.

In a particular embodiment, the isoxazole compound is isoxazole 9 (abbreviated as ISX9), which has an isoxazole cycloamide bond skeletal structure, and particularly it has a structure represented by formula (III).

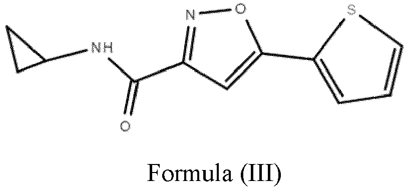

In a particular embodiment, isoxazole 9 or a derivative thereof has the structure represented by formula (I), 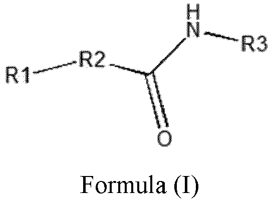 wherein R1 group may be any one selected from the group consisting of: thienyl, furanyl, pyrrolyl, phenyl and pyridyl; R2 group may be any one selected from the group consisting of: isoxazolyl, isothiazolyl, pyrazolyl, oxazoly, thiazolyl and imidazolyl, R3 group may be any one selected from the group consisting of: methyl, ethyl, cyclopropyl, cyclobutyl and cyclopentyl; wherein the linking site of R1 group is any carbon atom, the two linking sites of the R2 group are two meta-position carbon atoms, and the linking site of the R3 group is not limited.

In the method for inducing the transdifferentiation of non-neuronal cells into neurons by a using a composition comprising a myosin inhibitor, and an isoxazole and/or a derivative thereof according to the present application, the non-neuronal cells are cultured in an induction culture solution, followed by a mature culture solution, wherein the induction culture solution comprises a myosin inhibitor; and the mature culture solution comprises a myosin inhibitor, and an isoxazole compound and/or a derivative thereof. During the culture process, the cells have obvious neuron morphology with prominent cell bodies and obvious synapses; the positive rate of neuronal marker staining was 99.6%. Neural transcription factors were up-regulated, synapse-related gene expressions were up-regulated, and the transdifferentiated neurons were mainly GABAergic neurons. With the culture time from D0 to D45, the nerve-related genes are more and more approximate to those of the neurons, and from D14 to D45 the color depth of the cells is close to that of the neurons, indicating that the expression mode of the induced neurons is similar to that of the stem cell-derived neurons and the primary isolated neurons. The single-cell sequencing results show that the overall transcriptional level of neurons on day 30 and day 45 of the culture process is closer to that of the neurons isolated from the body. Compared with the cells obtained by the induction culture process of firstly culturing in an induction culture solution and subsequently culturing in a mature culture solution without Ble and ISX9, the neurons obtained by the induction culture method according to the present application have higher gene expression levels of neuronal markers. After the induction culture the cells were injected into the left hippocampus of mice. In the hippocampus at the side where the induced neurons were injected, GFP-positive induced neurons were observed, and the canonical neuronal markers MAP2 and NEUN were expressed. Compared with the cells obtained by culturing in a culture solution supplemented with a mixture of Ble and Ble-OB, and the cells obtained by culturing in a culture solution supplemented with Ble-OB, the neurons obtained by culturing in a culture solution supplemented with Ble have the highest gene expression levels of neuronal markers.

### EXAMPLES

### Example 1: Small Molecule Myosin II Inhibitor (-)-Blebbistatin Combined with Isoxazole 9 Efficiently Achieves Neuronal Transdifferentiation in Vitro

Taking a 10 cm vessel as an example (Corning, 430167), each vessel was coated with 3 mL of 20 µg/mL fibronectin solution (millipore, fc010, prepared with 1×PBS) for 6 hours. After removing the fibronectin solution, the human foreskin fibroblasts (HFF1y, Beijing Stem Cell Bank) were evenly inoculated at 2×10⁶ cells per vessel, culturing with basal medium (high glucose DMEM (Gibco, C12430500BT) plus 10% fetal bovine serum (Gibco, 16000-044)) for 12 hours. After removing the basal medium, the cells were washed with PBS.

The cell transformation kit of the present application was used for neuronal transdifferentiation, and the kit comprises the following induction culture solution and mature culture solution.

An induction culture solution was added to the HFF1y vessel after undergoing the above treatment to culture for 1-7 days. The induction culture solution was prepared by adding 25 µM Ble (MCE, HY-13441) to the N2B27 culture solution, wherein the N2B27 culture solution was prepared by firstly mixing DMEM/F12 (Gibco, 10565018) and Neurobasal (Gibco, 21103-049) at a ratio of 1:1, then adding N2 cell culture additive (100×, Gibco, 17502048), B27 cell culture additive (50×, Gibco, 17504044), β-mercaptoethanol (1000×, Gibco, 21985023), Glutamax (100×, Gibco, 35050-061), 1 µg/mL insulin (Roche, 11376497001), and penicillin-streptomycin (100×, gibco, REF 15140-122). After culturing in the induction culture solution for 1-7 days, the HFF1y cells show obvious neuronal morphology.

After the above induction culture for 1-7 days, a mature culture solution was added to HFF1y to culture for 7-45 days. The mature culture solution was prepared by adding 20ng/mL neurotrophin-3 (Peprotech, 450-03), 20ng/mL brain-derived neurotrophic factor (peprotech, 450-02), and 20ng/mL glial cell-derived neurotrophic factor (peprotech, 450-10), 10 µM forskolin (Stemgent, 04-0025), 20 µM Ble (MCE, HY-13441), and 30 µM ISX9 (MCE, HY-12323) into the above N2B27 culture solution.

To further illustrate the neuronal transdifferentiation effects of Ble and ISX9, the applicant provides Figs. 1A-1F.

A micrograph of HFF1y after culturing in high glucose DMEM plus 10% fetal bovine serum is shown in the left panel of Fig. 1A. A micrograph of the induced neurons after firstly culturing the above-mentioned HFF1y in an induction culture solution comprising Ble for 7 days, then culturing in a mature culture solution comprising Ble and ISX9 for 21 days, is shown in the right panel of Fig. 1A. As shown in the right panel of Fig. 1A, after 21 days of culture the cells show obvious neuronal morphology with prominent cell bodies and obvious synapses.

The cells in Fig. 1A (right) were stained for canonical neuronal markers, and the staining results are shown in Fig. 1B. Particularly, the neuronal markers include: TUJ1 (Covance, MRB-435P), MAP2 (santa cruz biotechnology, sc-20172), NF2000 (Abcam, ab4680) and NEUN (chemicon, MAB377).The above neuronal markers are all positive for staining, and the positive rate is close to 100%, particularly 99.6%.

Fig. 1C shows the gene expressions of neuronal markers of the cells obtained by firstly inducing the HFF1y in Fig. 1A (left) in an induction culture solution for 7 days, then culturing in a mature culture solution for 38 days. It can be seen from Fig. 1C that, the fibroblast marker FSP1 is down-regulated; the canonical neuronal markers such as GFAP, DCX, TUJ1, MAP2, NEUN are significantly up-regulated; at the same time, neural transcription factors such as ASCL1, BRN2, NEUROD1 are up-regulated, indicating the acquisition of neural fate for the induced cells; the up-regulation of synapse-related genes such as NEFH, PSD95, SYN1 and SYT lays the foundation for the function of the induced neurons; in addition, the up-regulation of gene expressions of PVALB, GAD and GABBR3 indicates that transdifferentiated neurons are mainly GABAergic neurons.

A heat map of the expression of neural-related genes during the induction of neurons is shown in Fig. 1D. D0 represents the initial cell state on day 0 of induction of human foreskin fibroblasts (HFF1y, Beijing Stem Cell Bank); h represents hours; D represents days; 3h, 6h, D1, D2, D7, D14, D30, D45 respectively represent different induction culture stages, i.e., the 3rd hour, the 6th hour, day 1, day 2 and day 7 (corresponding to the 3h, 6h, D1, D2, D7 in Fig. 1D, respectively) of induction of the HFF1y in Fig. 1A (left) in an induction culture solution, and day 7, day 23 and day 38 of the continuous culture in a mature culture solution (corresponding to D14, D30 and D45 in Fig. 1D, respectively); HNcDNA is primary human neurons; GABAN is human GABAergic neurons differentiated from pluripotent stem cells. The ordinate on the right side of the Fig. 1D is the gene name, the abscissa is the sample name, each small square with a color represents the level of the gene expression, and the upper left corner is the color scale. Each row represents the expression of each gene in different samples, and each column represents the expression of all genes in each sample. The dendrogram on the left represents the cluster analysis results of different genes from different samples. It can be clearly seen in Fig. 1D that, as the induction time from D0 to D45, the nerve-related genes of induced neurons are more and more approximate to those of the neurons, indicating that the expression mode of the induced neurons is similar to that of the stem cell-derived neurons and the primary isolated neurons.

Patch clamp experiments were performed on the induced neurons in Fig. 1A (right), and the results are shown in Fig. 1E. Patch clamp is an electrophysiological technique mainly used to detect the activity of cell membrane ion channels and induced action potentials. The electrophysiological results show that, the induced neurons have the sodium current, potassium current and induced action potential of mature neurons, indicating that the induced neurons have electrophysiological properties.

Fig. 1F shows the single-cell sequencing results during the whole process of firstly culturing the human foreskin fibroblasts (HFF1y, Beijing Stem Cell Bank) of Fig. 1A (left) in the induction culture solution of this example for 7 days, and then culturing them in the mature culture solution of this example for 38 days. Particularly, D0 is the initial cell state on day 0 of induction, and 3h, 6h, D1, D2, D7, D14, D30, and D45 respectively represent the 3^{rd} hour, 6^{th} hour, day 1, day 2, day 7, day 24, day 30, day 45 of the culture in this method, and "Neurons 1-6" are neurons isolated in vivo. The results of the analysis show that, the overall transcriptional level of neurons on day 30 and day 45 of the culture process is closer to that of isolated neurons in vivo.

### Comparative Example 1

With reference to the experimental operations of Example 1, the difference of this example lies in that: the HFF1y cells were firstly cultured in an induction culture solution for 7 days, and then cultured in a mature culture solution (compared with the mature culture solution in Example 1, the mature culture solution of this comparative example was not added with Ble and ISX9) for 38 days. The gene expressions of neuronal markers of the cells are shown in Fig. 1G. Fig. 1G also shows: the gene expressions of neuronal markers of the cells obtained by firstly culturing in the induction culture solution of Example 1 for 7 days, then culturing in the mature culture solution of Example 1 for 38 days; and the gene expressions of neuronal markers of the blank control cells. Fig. 1G shows the gene expressions of neuronal markers of the neurons obtained by the induction culture method according to the present application is higher.

### Example 2: Ble Combined with an Isoxazole Compound or a Derivative Thereof Efficiently Achieves Neuronal Transdifferentiation

A derivative of isoxazole compound ISX9, N-methyl-5-phenylisoxazole-3-carboxamide (abbreviated as ISX-PCA, 20-50µM, TCI, BD399148) was selected, wherein R1 group is benzene ring, the R3 group is methyl, and the obtained ISX-PCA has a structure represented by formula (IV). Neuronal transdifferentiation was performed according to the method of Example 1, except that ISX-PCA was used instead of ISX9.

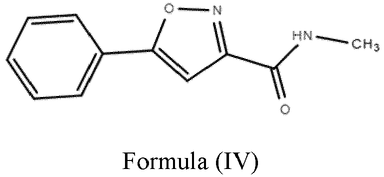

Fig. 2A shows a micrograph of HFF1y (left), and a micrograph of induced neurons (right) obtained by firstly culturing the HFF1y in an induction culture solution comprising Ble for 7 days, then continuously culturing the HFF1y in a mature culture solution comprising Ble and ISX-PCA for 21 days. As shown in Fig. 2A (right), the cells induced by combination of Ble and ISX-PCA have similar morphology to the cells in Fig. 1A (right), and they have obvious neuronal morphology, prominent cell bodies, and obvious synapses.

The gene expressions of canonical neuronal markers were further detected on the induced neurons in Fig. 2A (right), and the results are shown in Fig. 2B, particularly, canonical neuronal markers such as GFAP, DCX, TUBB3, MAP2, NEUN, MAPT, and NEFH are significantly up-regulated; at the same time, neural transcription factors such as ASCL1, BRN2, and NEUROD1 are up-regulated, indicating that the transdifferentiated cells acquire neural fate.

### Example 3: Ble Combined with an Isoxazoles and a Derivative Thereof Promotes Neuronal Transdifferentiation of Human Fibroblasts in Vivo

Taking a 10 cm vessel as an example (Corning, 430167), each vessel was coated with 3 mL of 20 µg/mL fibronectin solution (millipore, fc010, prepared with 1×PBS) for 6 hours. After removing the fibronectin solution, the human foreskin fibroblasts (HFF13y-GFP, Beijing Stem Cell Bank) were evenly inoculated at 2×10⁶ cells per vessel, culturing with basal medium (high glucose DMEM (Gibco, C12430500BT) plus 10% fetal bovine serum (Gibco, 16000-044)) for 12 hours. After removing the basal medium, the cells were washed with PBS.

An induction culture solution of Example 1 was added to the HFF 13y-GFP culture vessel after undergoing the above treatment to culture for 7 days, and the cells were continuously cultured in a mature culture solution of Example 1 for 7 days. Then the cells were injected into the left hippocampus of 5 week-old immunodeficiency (SCID) mice at a cell number of 2.5×10⁵ cells/mouse, marking the transplanted cells with green fluorescent protein (GFP); and then the mice were intraperitoneally injected at a dose of 3 mg/kg Ble + 10 mg/kg ISX9 every day for 14 consecutive days, wherein the injection solvent consists of dimethyl sulfoxide (DMSO) (2% in final volume) + PEG400 (40% in final volume) + Tween 80 (2% in final volume) + sterile water. 14 days later, the hippocampus was taken for paraffin section staining to detect the expression of GFP and canonical neuronal markers. The results are shown in Fig. 3, in which, counting from top to bottom, the first and second rows are the control group, i.e., the staining results of the neuronal marker protein MAP2 (the first row), NEUN (the second row) and GFP (derived from the transplanted induced cells) after induction in vivo on the side of the brain without the transplanted cells; the third and fourth rows in Fig. 3 are staining results of the neuronal marker protein MAP2 (the third row), NEUN (the fourth row) and GFP (derived from the transplanted fibroblasts to be induced) after induction in vivo on the side of the brain of the same mouse with the transplanted cells. Merge represents the overlapped panel, the third and fourth rows show that there are GFP-positive cells expressing MAP2 (in the overlapped panel in the third row, determining by signal co-localization, the cells having GFP signal also have MAP2 signal) and NEUN (in the overlapped panel in the fourth row, determining by signal co-localization, the cells having GFP signal also have NEUN signal), indicating that the transplanted GFP cells become neurons after induction in vivo. It can be seen from Fig. 3 that, in the hippocampus on the side where the induced neurons are injected, GFP-positive induced neurons can be observed, and they express the canonical neuronal markers MAP2 and NEUN.

### Example 4: Ble Promotes Neuron Morphogenesis and Up-regulation of Genes Related to Neural Fate

Taking a 10 cm vessel as an example (Corning, 430167), each vessel was coated with 3 mL of 20 µg/mL fibronectin solution (millipore, fc010, prepared with 1 ×PBS) for 6 hours. After removing the fibronectin solution, the HFF1y were evenly inoculated at 2×10⁶ cells per vessel, culturing with basal medium (high glucose DMEM (Gibco, C12430500BT) plus 10% fetal bovine serum (Gibco, 16000-044)) for 12 hours. After removing the basal medium, the cells were washed with PBS.

An induction culture solution of Example 1 was added to the HFF1y vessel after undergoing the above treatment to culture for 1-7 days, the morphological changes of the cells are shown in Fig. 4A. After induction, the cells have obvious neuronal morphology, small and round cell bodies, and rich and long synapses.

RNA-seq, GO cluster analysis were performed on the above cells, and the results are shown in Fig. 4B, wherein the length of red bar represents the negative log value (p<0.05) of the significant p-value enriched in this process. Fig. 4B shows that in the neural induction culture system comprising only Ble, the up-regulated genes in induced cells were enriched in the neuronal fate-related pathways, which indicated that Ble alone may promote neuronal morphogenesis and the up-regulation of neural fate-related genes.

### Example 5: Isoxazole Compound and a Derivative Thereof Potently Promote Neural Fate Transformation at Transcriptional Level

With reference to the experimental operations of Example 1, the difference of this example lies in that: the HFF1y cells were cultured in an induction culture solution (compared to the induction culture solution in Example 1, the induction culture solution of this example does not comprise Ble) for 7 days, and then continuously cultured in a mature culture solution (compared to the mature culture solution in Example 1, the mature culture solution of this example does not comprise Ble) for 7 days. The morphological changes of the cells are shown in Fig. 5A (right panel), and Fig. 5A (left panel) shows the HFF1y; and it can be seen from a comparison of the left and right panels that, the induced cells do not appear neuron-like morphology.

Gene expressions of canonical neuronal markers were further examined for Fig. 5A (right).The results are shown in Fig. 5B, the fibroblast marker FSP1 is significantly down-regulated, and the expressions of canonical neuronal markers such as GFAP, TUJ1, MAP2, MAPT, STMN1 and NCAM, and synapse-related proteins such as NEFH, PSD95, SYN1 and SYT are significantly up-regulated. At the same time, neural transcription factors such as ASCL1, BRN2 and NEUROD1 are up-regulated, indicating that the transdifferentiated cells acquire neural fate.

According to the results of Example 4 and Example 5, it can be inferred that in the process of promoting the efficient transformation of fibroblasts into neurons by a combination of Ble and an isoxazole or a derivative thereof, Ble is responsible for neuromorphogenesis and initiates the transformation of neural fate, while the isoxazole or a derivative thereof potently promotes complete transdifferentiation of neuronal fate.

### Example 6: Ble Combined with an Isoxazole or a Derivative Thereof has a Potent Neuron-Inducing Effect

To further verify the potent neuron-inducing effect of Ble + an isoxazole or a derivative thereof. The HFF1y cells as shown in Fig. 5A (left panel) were firstly cultured in an induction culture solution of Example 1 for 7 days, then continuously cultured them in a neural mature culture solution without forskolin, or a neural mature culture solution without forskolin, neurotrophin-3, brain-derived neurotrophic factor and glial cell-derived neurotrophic factor (as compared with the mature culture solution of Example 1) for 7 days; and the gene expressions of canonical neuronal markers were detected. The results are shown in Fig. 6, for the cells treated with a neural mature culture solution without forskolin, or a neural mature culture solution without forskolin, and above three neurotrophic factors, the canonical neuronal markers such as DCX, TUBB3, MAP2, NEUN, MAPT and STMN1, and synapse-related proteins such as NEFH, SYT1, SYN1 and PSD95 are significantly up-regulated. At the same time, neural transcription factors such as ASCL1, BRN2 and NEUROD1 are up-regulated, indicating that the transdifferentiated cells acquire neural fate.

### Example 7: Ble Combined with an Isoxazole or a Derivative Thereof Achieves Efficient Transdifferentiation of Mouse Astrocytes into Neurons

Astrocytes are one of the main cells in the brain environment, and are involved in the physiological functions of the brain. Transdifferentiation of astrocytes into neurons has important guidance for the treatment of neurodegenerative diseases such as Parkinson's and Alzheimer's disease.

With reference to the experimental operations of Example 1, the difference of this example lies in that: the HFF1y cells were replaced with astrocytes from mouse cerebral cortex, and the astrocytes from mouse cerebral cortex were treated. The mouse astrocytes were firstly cultured in an induction culture solution of Example 1 for 1 day, and then cultured in a mature culture solution of Example 1 for 13 days; after culturing for 13 days, the cell morphology and the staining of canonical neuronal markers are shown in Fig. 7A. The cell morphology of the induced cell from mouse astrocytes is similar to that of neurons; the induced cells have small and round cell bodies, abundant synapses, and express canonical neuronal markers such as TUJ1, MAP2 and NEUN.

The gene expressions of neuronal markers of the induced cells of Fig. 7A are shown in Fig. 7B. The results show that canonical neuronal markers such as DCX, TUBB3, MAP2, NEUN, MAPT and NEFH are significantly up-regulated; the expression of postsynaptic density protein PSD95 is up-regulated; meanwhile, neural transcription factors such as ASCL1, BRN2 and MYTL1 are up-regulated, indicating that the transdifferentiated cells acquire neural fate.

### Example 8

With reference to the experimental operations of Example 1, the difference of this example lies in that: the HFF1y cells were cultured in an induction culture solution for 7 days, and then continuously cultured in a mature culture solution for 7 days.

### Example 9

With reference to the experimental operations of Example 1, the difference of this example lies in that: the HFF1y cells were cultured in an induction culture solution (compared with the induction culture solution of Example 1, Ble-OB instead of Ble was used in the induction culture solution of this example) for 7 days, and then continuously cultured in a mature culture solution (compared with the mature culture solution of Example 1, Ble-OB instead of Ble was used in the mature culture solution of this example) for 7 days.

### Example 10

With reference to the experimental operations of Example 1, the difference of this example lies in that: the HFF1y cells were cultured in an induction culture solution (compared with the induction culture solution of Example 1, Ble-OB instead of Ble was used in the induction culture solution of this example) for 7 days, and then continuously cultured in a mature culture solution (compared with the mature culture solution of Example 1, ISX-PCA instead of ISX9, and Ble-OB instead of Ble were used in the mature culture solution of this example) for 7 days.

### Example 11

With reference to the experimental operations of Example 1, the difference of this example lies in that: the HFF1y cells were cultured in an induction culture solution (compared with the induction culture solution of Example 1, a mixture of Ble-OB and Ble at a ratio of 1:1 instead of Ble was used in the induction culture solution of this example; wherein the total concentration of Ble-OB and Ble in the induction culture solution of this example is equal to the concentration of Ble in the induction culture solution of Example 1) for 7 days, and then continuously cultured in a mature culture solution (compared with the mature culture solution of Example 1, a mixture of Ble-OB and Ble at a ratio of 1:1 instead of Ble was used; wherein the total concentration of Ble-OB and Ble in the mature culture solution of this example is equal to the concentration of Ble in the mature culture solution of Example 1) for 7 days.

### Example 12

With reference to the experimental operations of Example 1, the difference of this example lies in that: the HFF1y cells were cultured in an induction culture solution (compared with the induction culture solution of Example 1, Ble-OB instead of Ble was used in the induction culture solution of this example) for 7 days, and then continuously cultured in a mature culture solution (compared with the mature culture solution of Example 1, Ble-OB instead of Ble, and a mixture of ISX9 and ISX-PCAat a ratio of 1:1 instead of ISX9 was used; wherein the total concentration of ISX9 and ISX-PCA in the mature culture solution of this example is equal to the concentration of ISX9 in the mature culture solution of Example 1) for 7 days.

### Comparative Example 2

With reference to the experimental operations of Comparative Example 1, the difference of this example lies in that: the HFF1y cells were cultured in an induction culture solution for 7 days, and then continuously cultured in a mature culture solution for 7 days.

The gene expressions of neuronal markers (MAPT and MAP2) of neurons obtained in Examples 8-12 and Comparative Example 2 are shown in Fig. 8. It can be seen that, compared with the cells obtained by induction culture in a culture solution without an isoxazole and/or a derivative thereof, the gene expression levels of the neuronal markers of the neurons obtained by inducing and culturing in a culture solution comprising an isoxazole and/or a derivative thereof are obviously improved. In addition, with a comparison of the gene expression levels of neuronal markers in neurons obtained from culture solutions respectively comprising: Ble, a mixture of Ble and Ble-OB, and Ble-OB, the gene expression level of neuronal markers of the neurons obtained from a culture solution comprising Ble is the highest, followed by a culture solution comprising a mixture of Ble and Ble-OB, and the third is a culture solution comprising Ble-OB.

The above are only preferred examples of the present application, and are not intended to limit the present application in other forms. Any person skilled in the art may use the technical content disclosed above to make changes or modifications to obtain equivalent examples with equivalent changes. However, any simple modifications, equivalent changes and modifications made to the above examples according to the technical essence of the present application without departing from the content of the technical solutions of the present application still fall within the protection scope of the technical solutions of the present application.

## Claims

1. A composition for inducing cell transdifferentiation, comprising:
a myosin inhibitor, and
an isoxazole compound and/or a derivative thereof.

2. Use of a composition comprising a myosin inhibitor, and an isoxazole compound and/or a derivative thereof in inducing cell transdifferentiation; preferably, the transdifferentiation is to induce the transdifferentiation of non-neuronal cells into neurons.

3. Use of a composition comprising a myosin inhibitor, and an isoxazole compound and/or a derivative thereof in the preparation of a medicament for treating a neurodegenerative disease.

4. A method for inducing transdifferentiation of non-neuronal cells into neurons, comprising treating the non-neuronal cells with a myosin inhibitor, and an isoxazole compound and/or a derivative thereof.

5. The method according to claim 4, wherein it comprises: culturing the non-neuronal cells in an induction culture solution for 1-7 days, and then culturing them in a mature culture solution for 7-45 days, preferably 21-45 days.

6. A culture medium for inducing transdifferentiation of non-neuronal cells into neurons, comprising an induction culture solution and a mature culture solution.

7. The method according to claim 5 or the culture medium according to claim 6, wherein the induction culture solution comprises a myosin inhibitor;
preferably, the induction culture solution further comprises N2B27 culture solution, wherein the N2B27 culture solution is prepared by firstly mixing DMEM/F12 and Neurobasal at a ratio of 1:1, and then adding N2 cell culture additive, B27 cell culture additive, β-mercaptoethanol, Glutamax, insulin and penicillin-streptomycin.

8. The method according to claim 5 or the culture medium according to claim 6 or 7, wherein the mature culture solution comprises a myosin inhibitor, and an isoxazole compound and/or a derivative thereof;
preferably, the mature culture solution comprises: a myosin inhibitor, an isoxazole compound and/or a derivative thereof, N2B27 culture solution, neurotrophic factor and forskolin; wherein the N2B27 culture solution is prepared by firstly mixing DMEM/F12 and Neurobasal at a ratio of 1:1, and then adding N2 cell culture additive, B27 cell culture additive, β-mercaptoethanol, Glutamax, insulin and penicillin-streptomycin; preferably, the neurotrophic factor comprises: neurotrophin-3, brain-derived neurotrophic factor and glial cell-derived neurotrophic factor;
preferably, the mature culture solution comprises: a myosin inhibitor, an isoxazole compound and/or a derivative thereof, and the N2B27 culture solution;
preferably, the mature culture solution consists of: a myosin inhibitor, an isoxazole compound and/or a derivative thereof, and the N2B27 culture solution;
preferably, the mature culture solution comprises: a myosin inhibitor, an isoxazole compound and/or a derivative thereof, the N2B27 culture solution, neurotrophin-3, brain-derived neurotrophic factor and glial cell-derived neurotrophic factor;
preferably, the mature culture solution consists of: a myosin inhibitor, an isoxazole compound and/or a derivative thereof, the N2B27 culture solution, neurotrophin-3, brain-derived neurotrophic factor and glial cell-derived neurotrophic factor.

9. A method for transdifferentiating non-neuronal cells into neurons in a subject, comprising administering to the subject an effective amount of a myosin inhibitor, and an isoxazole compound and/or a derivative thereof;
preferably, the method comprises: administering to the subject an effective amount of a myosin inhibitor, and an isoxazole compound and/or a derivative thereof by intraperitoneal injection;
preferably, the method comprises: culturing the non-neuronal cells in an induction culture solution and a mature culture solution in sequence, then injecting the cultured non-neuronal cells into the body, and finally administering to the subject an effective amount of a myosin inhibitor, and an isoxazole compound and/or a derivative thereof by intraperitoneal injection;
preferably, the method comprises: culturing the non-neuronal cells in an induction culture solution for 1-7 days and subsequently in a mature culture solution for 5-10 days, then injecting the cultured non-neuronal cells into the body, and finally administering to the subject an effective amount of a myosin inhibitor, and an isoxazole compound and/or a derivative thereof by intraperitoneal injection for 14 or more consecutive days.

10. A method for treating a neurodegenerative disease in a subject, comprising administering to the subject an effective amount of a myosin inhibitor, and an isoxazole compound and/or a derivative thereof.

11. The use according to claim 3 or the method according to claim 10, wherein the neurodegenerative disease includes: Parkinson's disease, Alzheimer's disease, amyotrophic lateral sclerosis, Huntington's disease, spinocerebellar ataxia, epilepsy, stroke, brain injury, and spinal cord injury.

12. Neurons obtained by the method according to any one of claims 4-5 and 7-8.

13. An article of manufacture or kit for transdifferentiating non-neuronal cells into neurons, wherein the article of manufacture or kit comprises an induction culture solution and a mature culture solution, wherein
the induction culture solution comprises a myosin inhibitor;
the mature culture solution comprises a myosin inhibitor, and an isoxazole compound and/or a derivative thereof.

14. Use of a myosin inhibitor in the promotion of neuronal morphogenesis and the initiation of neural fate.

15. Use of an isoxazole compound or a derivative thereof in the promotion of efficient expression of neuron genes.

16. The composition according to claim 1, the use according to any one of claims 2-3, 11 and 14, the method according to any one of claims 4-5 and 7-11, the culture medium according to claim 7 or 8, the neurons according to claim 12, or the article of manufacture or kit according to claim 13, wherein the myosin inhibitor is (-)-Blebbistatin, and/or (-)-Blebbistatin O-Benzoate.

17. The composition according to claim 1 or 16, the use according to any one of claims 2-3, 11 and 15-16, the method according to any one of claims 4-5, 7-11 and 16, the culture medium according to claims 8 or 16, the neurons according to claim 12 or 16, or the article of manufacture or kit according to claim 13 or 16, wherein the isoxazole compound and/or a derivative thereof has a structure represented by the following formula (I), 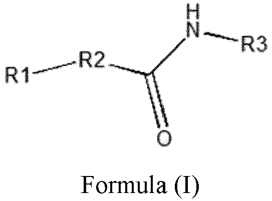 in formula (I), R1 group is any one selected from the group consisting of: thienyl, furanyl, pyrrolyl, phenyl and pyridyl; R2 group is any one selected from the group consisting of: isoxazolyl, isothiazolyl, pyrazolyl, oxazoly, thiazolyl and imidazolyl, R3 group is any one selected from the group consisting of: methyl, ethyl, cyclopropyl, cyclobutyl and cyclopentyl; wherein the linking site of the R1 group is any carbon atom, the two linking sites of the R2 group are two meta-position carbon atoms, and the linking site of the R3 group is any carbon atom.

18. The composition, use, method, culture medium, neurons, article of manufacture or kit according to claim 17, wherein the isoxazole compound and/or a derivative thereof is any one or two or more selected from the group consisting of: isoxazole 9 (ISX9), N-methyl-5-phenylisoxazole-3-carboxamide (ISX-PCA), N,5-dimethylisoxazole-3-carboxamide, N-methyl-5-(pyridin-4-yl)isoxazole-3-carboxamide, N-methyl-5-phenylisothiazole-3-carboxamide, N-methyl-5-phenyl-1H-pyrazole-3-carboxamide, N-methyl-2-phenyloxazole-4-carboxamide, N-methyl-2-phenylthiazole-4-carboxamide, N-methyl-2-phenyl-1H-imidazole-4-carboxamide, N-methyl-5-(thiophen-2-yl)isoxazole-3-carboxamide, 5-(furan-2-yl)-N-methylisoxazole-3-carboxamide, N-methyl-2-(thiophen-2-yl)-1,3-thiazole-4-carboxamide.

19. The use according to any one of claims 2 and 16-18, the method according to any one of claims 4-5, 7-9 and 16-18, the culture medium according to any one of claims 6-8 and 16-18, the neurons of any one of claims 12 and 16-18, or the article of manufacture or kit according to any one of claims 13 and 16-18, wherein the non-neuronal cells are fibroblasts or astrocytes.
